# DEMANDE DE BREVET EUROPEEN

(11) **EP 0 770 686 A1**
(43) Date de publication de la demande: **02.05.1997**
(21) Numéro de dépôt: 95202889.2
(22) Date de dépôt: 25.10.1995
(51) Int. Cl.: C12P 11/00, A23L 1/226, C07D 307/38

(54) **Procédé de préparation de thiols**

(71) Demandeur: SOCIETE DES PRODUITS NESTLE S.A., 1800 Vevey (CH)
(72) Inventeur: Huynh-Ba, Tuong, CH-1009 Pully (CH); Jaeger, Daniel, CH-1064 St-Cierges (CH); Matthey-Doret, Walter, CH-1092 Belmont S/Lausanne (CH)
(74) Mandataire: Archambault, Jean

(57) **Abrégé**

On prépare des thiols, notamment des thiols naturels par bioconversion en mettant en présence un S-conjugué de cystéine et un micro-organisme utilisable en alimentation ayant une activité enzymatique de type béta-C-S-lyase.

Les micro-organismes mis en oeuvre sont de préférence les levures et les champignons filamenteux.

Le produit obtenu peut être utilisé dans un aliment, tel quel, dans une composition aromatisante ou comme renforçateur d'arôme.

## Description

L'invention se rapporte à la préparation de thiols, notamment de thiols naturels aromatisants par bioconversion.

Les thiols sont parmi les substances volatiles contenant du soufre celles que l'on estime responsables de la note viandée de différentes viandes, par exemple de poulet, de boeuf et de porc, ou qui sont des composants de l'arôme de café.

Les arômes de viandes sont généralement produits par réaction thermique entre un sucre réducteur et un acide aminé en présence d'un composé contenant du soufre comme source de sulfure, par exemple la cystéine, à température élevée pendant une certaine durée, le cas échéant en présence de graisse. Une telle réaction fournit un mélange complexe de substances aromatiques entrant dans la composition de l'arôme de viande.

Le but de la présente invention est de mettre à disposition des thiols naturels susceptibles de servir d'ingrédients d'aromatisation des aliments, obtenus par bioconversion.

On connait, par exemple de US-A-5.182.194, l'aptitude de certains micro-organismes non utilisables en alimentation à fournir une enzyme béta-C-S-lyase susceptible de produire des thiols à partir de S-conjugués de cystéine.

Le procédé selon l'invention est caractérisé par le fait que l'on met en présence un S-conjugué de cystéine de formule: 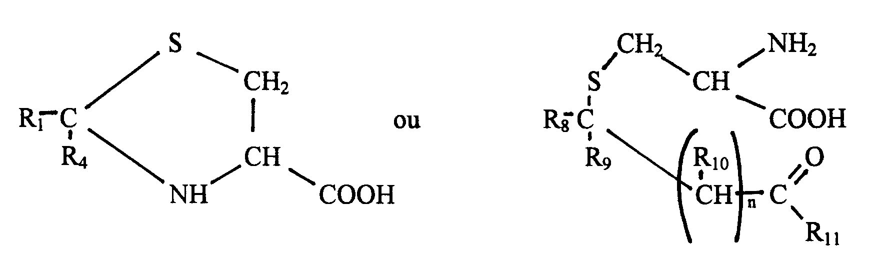 dans laquelle:
R₁ est H, C₁₋₇-alkyl, linéaire ou branché, phényl, benzyl, 2-R₂-2-R₃-vinyl, 5-R₂-furan-2-yl, 5-R₂-thiofuran-2-yl,
R₂ et R₃ sont semblables ou différents et représentent H ou CH₃,
R₄ est H, ou forme avec R₁ un groupe 2-R₅-5-R₆-tétrahydrofuran-3-ylidène, 2-R₅-5-R₆-2,3-dihydrofuran-3-ylidène, dans lequel R₅ et R₆ sont semblables ou différents et représentent H ou CH₃,
ou R₄ est un groupe CH(OH)R₇ et R₇ représente H, C₁₋₄-alkyl ou phényl,
R₈, R₉ et R₁₀ sont semblables ou différents et représentent H ou C₁₋₄-alkyl,
R₁₁ représente H, C₁₋₄-alkyl, phényl, C₁₋₄-alkoxy ou C₁₋₄-alkylamino et n est 0 ou 1,
et un micro-organisme utilisable en alimentation ayant une activité enzymatique de type béta-C-S-lyase.

Dans le contexte de l'invention, un radical R₁ alkyl en C₁₋₇ est de préférence méthyl, éthyl, n-propyl, isopropyl, n-butyl, isobutyl, sec-butyl, n-hexyl ou n-heptyl.

Un radical R₈ à R₁₀ est de préférence H ou méthyl et R₁₁ méthyl, éthyl, phényl, méthoxy, éthoxy, méthylamino ou éthylamino.

Sous l'action de la béta-C-S-lyase, le radical cystéyl du composé conjugué est scindé en position béta du groupe carboxyl final, ce qui conduit au thiol correspondant.

Lorsque n est 0, le composé formé par l'action de la béta-C-S-lyase est ainsi un alpha-mercapto aldéhyde une alpha-mercaptocétone, un alpha-mercaptoester ou une alpha-mercaptoamide.

Lorsque n est 1, le composé formé par l'action de la béta-C-S-lyase est ainsi un béta-mercapto aldéhyde, une béta-mercaptocétone, un béta-mercaptoester ou une béta-mercaptoamide.

Les composés préférés libérés par la béta-C-S-lyase sont le furfuryl thiol (FFT), le benzyl thiol, le 5-méthylfurfuryl thiol, le 2-méthylfuran-3-thiol, le 2,5-diméthylfuran-3-thiol, le 2-méthyl-tétrahydrofuran-3-thiol et le 3-méthyl-2-butène-1-thiol.

Ces composés contribuent de manière significative à la génération de l'arôme de type viande, de poulet, de boeuf et de porc et également de café en ce qui concerne les furanthiols et le 3-méthyl-2-butène-1-thiol.

On a constaté que les composés préférés précédents peuvent être métabolisés en partie en sulfure de furfuryl-méthyl (Me-FFT), S-furfurylthio acétate (Ac-FFT)et bis-furfuryl disulfure (Di-FFT). Ces métabolites possèdent également une note viandée caractéristique et peuvent contribuer également à la formation de l'arôme.

Selon l'invention, les micro-organismes entrant en ligne de compte sont tous ceux qui sont utilisables en alimentation et susceptibles de posséder une activité enzymatique de type béta-C-S-lyase.

Ce sont, de préférence, des levures et des champignons filamenteux ou moisissures. Parmi les levures, on peut citer particulièrement la levure boulangère, Saccharomyces cerevisae, mais également, par exemple, la levure de bière, les levures Candida versatilis, Debaromyces hansenii, Saccharomyces bayanus, Saccaromyces rouxii. Parmi les champignons filamenteux, on peut citer, par exemple, Aspergillus oryzae, Penicillium roqueforti, Penicillium camemberti.

Les conjugués de cystéine susceptibles de servir de substrat dans la bioconversion selon l'invention peuvent être obtenus aisément par
réaction d'une aldéhyde ou d'une cétone de formule R₁ -CHO ou R₁, R₄-CO ou d'une cétone, d'un ester ou d'une amide alpha, béta- insaturés de formule CR₈,R₉ =(CR₁₀)ₙCOR₁₁ , dans lesquelles R₁ à R₁₁ ont les significations précédentes, avec la cystéine en milieu hydro-alcoolique et à la température ambiante.

La réaction d'incubation avec le micro-organisme a lieu dans des conditions d'activation de l'enzyme C-S-lyase du micro-organisme. Ce peut être dans des conditions d'anaérobie, soit d'aérobie, de préférence en anaérobie pendant 5 à 72 h et de préférence pendant 12 à 48 h et à pH 6-9, et de préférence à pH 7-8, sous agitation moyenne à forte et à une température de 20 à 50° C et de préférence autour d'environ 30° C. La concentration du substrat, c'est à dire du conjugué dans le bouillon est de 1 à 100 mMol et de préférence de 20 à 40 mMol.

En particulier dans le cas de la levure boulangère, on préfère opérer dans des conditions d'anaérobie, par exemple sous gaz inerte tel que l'azote, de manière à minimiser la génération de métabolites de la levure boulangère elle-même et de produits de transformation, par exemple par oxydation, de tels métabolites et donc d'orienter la fermentation vers la production de thiols.
La levure boulangère peut être mise en oeuvre sous forme de crème ou d'extrait. Elle est de préférence fraîche, particulièrement de 0 à 18 j, et avantageusement de 0 à 8 j, conservée au réfrigérateur.

L'incubation peut se faire par addition du substrat en une ou plusieurs fois, et de préférence de manière progressive, en tenant compte de la cinétique de la réaction enzymatique, de sorte que ce dernier n'inhibe pas l'activité de l'enzyme. Dans le même ordre d'idées, on peut prévoir, par exemple d'augmenter la quantité de substrat et d'extraire les thiols volatils au fûr et à mesure de leur production, de manière à augmenter le rendement et la vitesse de réaction de la lyse. A cet effet, on peut par exemple fixer les composés aromatiques volatils sur un support, par exemple une résine, et les en éluer ensuite. On peut également procéder à leur extraction durant l'incubation dans un milieu biphasique à l'aide d'un solvant non miscible à l'eau, de préférence de qualité alimentaire, par exemple le pentane ou l'hexane.

Après l'incubation, on peut extraire les thiols avec un solvant, de préférence de qualité alimentaire, par exemple le pentane ou l'hexane et les purifier par les méthodes classiques connues par l'homme du métier.

En variante, on peut extraire les thiols formés dans le surnageant que l'on peut séparer, par exemple par centrifugation, puis le cas échéant concentrer et sécher, par exemple par pulvérisation ou lyophilisation en présence d'un support solide, par exemple de maltodextrine. Le séchage a lieu dans des conditions modérées, par exemple à température < à 70° C sous vide.

L'invention concerne également l'utilisation du produit de la réaction précédente contenant les thiols et leurs métabolites dans un aliment, tel quel, dans une composition aromatisante ou comme renforçateur d'arôme.

De tels arômes peuvent être incorporés dans des aliments destinés à la nourriture humaine ou animale. Dans le présent exposé, on entend par micro-organisme utilisable en alimentation, tout micro-organisme de qualité alimentaire, que ce soit en alimentation humaine ou animale.

Les exemples ci-après illustrent l'invention. Dans ceux-ci, les pourcentages et parties sont en poids, sauf indication contraire.

### Exemples 1-4

### 1.1. Préparation de crème de levure boulangère

On clarifie de la crème de levure boulangère du commerce, Saccharomyces cerevisae à 22-28 % de matière sèche par centrifugation et on élimine le surnageant. On mélange ensuite le culot avec une solution aqueuse à 0,1 M de tampon phosphate de pH 7,5.

### 1.2. Préparation du conjugué cystéine-furfural ou acide 2-(2-furyl)-1,3-thiazolidine-4-carboxylique

On dissout 7,7 g (63,6 mmol) de cystéine dans 200 ml d'une solution aqueuse à 40 % d'éthanol dans un réacteur. On ajoute ensuite goutte à goutte une solution de 7 g (72,9 mmol) de furfural dnans 20 ml d'éthanol sous agitation. On laisse la solution jaune claire sous agitation et on constate l'apparition d'un précipité au bout d'1 h. Après avoir placé le réacteur dans un bain de glace pendant 1 h, on sépare le précipité par filtration, on le lave dans 2x 50 ml d'éthanol et on le sèche sous pression réduite. On obtient ainsi 10,95 g d'une poudre blanchâtre de conjugué cystéine-furfural avec un rendement de 86,5 %.

La structure du composé, vérifiée par spectrographie de masse à impact d'électron (MS/EI) et par résonance magnétique nucléaire du proton dans le diméthyl sulfoxyde (¹ H-NMR) montre la présence de deux diastéréoisomères cis/trans dans le rapport 1 : 2.

### 1.3. Incubation

On place alors 100 ml de la crème de cellules entières de levure boulangère précédente dans un réacteur équipé d'une électrode à pH, d'un réfrigérant et d'un agitateur magnétique tournant à 600 t/min. Le réacteur est placé dans un bain d'huile thermostaté à 30° C. Le réacteur est relié à un pH-stat maintenant le pH à la valeur choisie (6,9 ou 8) par adjonction d'une solution aqueuse 2N d'hydroxyde de sodium. Dans le cas d'un essai en anaérobie, on fait barboter de l'azote dans la suspension pendant 15 min. au moyen d'un tube à la sortie du réfrigérant et on maintient le réacteur sous azote pendant la durée de l'incubation. On y ajoute ensuite 0,4 g (2 mmol), respectivement 0,8 g (4 mmol) d'acide 2-(2-furyl)-1,3-thiazolidine-4-carboxylique. On prend des échantillons de 5 ml à différents temps d'incubation.

### 1.4. Analyse des résultats de la bioconversion

Après avoir ajusté le pH de l'échantillon à 4 avec une solution aqueuse 2N d'acide chlorhydrique, on y ajoute 500 microlitre d'une solution de benzyl thiol dans le pentane (2000 ppm., partie par million) en tant que standard interne. Après addition de 3 g de chlorure de sodium, on extrait l'échantillon avec 3x 15 ml d'éther diéthylique, on sépare les extraits du milieu réactionnel par centrifugation (15 min., 5000 t/min), on les combine, on les sèche sur sulfate de sodium, on les concentre dans une colonne de Vigreux en un volume de 2 ml et on entrepose la solution concentrée obtenue dans un congélateur à - 20° C jusqu'à l'analyse.

L'analyse par chromatographie gazeuse est effectuée sur chromatographe Carlo Erba Mega 2 équipé d'un injecteur à froid et d'un détecteur d'ionisation de flamme (GC-FID). On utilise un détecteur photométrique de flamme (GC-FPD) pour les composés soufrés. Les colonnes capillaires sont DB-5, DB-1701, DB-Wax et DB-FFAP, 30 m x 0,32 mm, épaisseur de film 0,25 micron, J&W Scientific, Folsom, USA. Le gaz vecteur est l'hélium (65 kPa) avec complément d'azote (40 kPa) pour la GC-FID. On calcule les indices de rétention (RI) par interpolation linéaire.

Les analyses ont été confirmées par l'établissement de spectres produits par couplage d'un chromatographe gazeux à un spectrographe de masse à impact d'électron (GC/EI-MS, HP 5890/HP 5971) dans les mêmes conditions opératoires que pour GC-FID.

Les résultats pour les composés soufrés principaux sont indiqués dans le tableau 1 relatif à l'incubation aérobie et dans le tableau 2 concernant l'incubation anaérobie ci-après.

**Tableau 1**

| **Composé** | **RI (DB- Wax), FID** | **RI (DB- Wax), FPD** | **RI (FFAP), FPD** | **RI (DB5), FPD** | **RI (DB- 1701), FPD** |
|---|---|---|---|---|---|
| S-Méthyl thioacétate | 1037 | 1039 | 1048 | - | - |
| S-Ethyl thioacétate | 1087 | 1088 | 1090 | - | - |
| Acide thioacétique | - | 1148 | - | - | 713 |
| Diéthyl disulfure | 1208 | 1192 | - | - | - |
| 2-Thio-1-éthanol | - | 1502 | 1500 | - | - |
| S-Furfuryl-thio thioacétate | 2215 | 2217 | - | - | - |
| FFT | 1434 | 1438 | 1437 | 908 | 1000 |
| Me-FFT | 1486 | 1487 | 1487 | 998 | 1091 |
| Ac-FFT | 1768 | 1769 | 1770 | 1159 | 1276 |
| Di-FFT | 2570 | 2570 | 2602 | 1690 | 1864 |

**Tableau 2**

| **Composé** | **RI (DB- Wax), FID** | **RI (DB- Wax), FPD** | **RI (FFAP), FPD** | **RI (DB5), FPD** | **RI (DB- 1701), FPD** |
|---|---|---|---|---|---|
| FFT | 1435 | 1440 | 1438 | 911 | 1000 |
| Me-FFT | 1487 | 1487 | 1487 | 998 | 1091 |
| Ac-FFT | 1768 | 1768 | 1769 | 1159 | 1276 |
| Di-FFT | - | 2570 | 2600 | 1689 | 1864 |

On constate que l'incubation aérobie produit d'autres composés soufrés en plus de ceux recherchés (FFT, Me-FFT, Ac-FFT et Di-FFT). Ces autres composés résultent probablement de la métabolisation de la cystéine.

L'incubation anaérobie produit principalement les composés recherchés.

Le tableau 3 ci-après donne le rendement en FFT, calculé en % du conjugué de départ en fonction des conditions d'incubation. Dans les exemples 1-4, la levure mise en oeuvre est âgée de 4 j.

**Tableau 3**

| **Exemple** | **1** | **2** | **3** | **4** |
|---|---|---|---|---|
| Aérobie | + | - | - | - |
| Anaérobie | - | + | + | + |
| pH | 6,9 | 6,9 | 6,9 | 8 |
| Concentration du substrat (mM) | 20 | 20 | 40 | 20 |

| **Rendement en FFT (%) après (j)** | | | | |
|---|---|---|---|---|
| 1 | 25,4 | 14 | 2,2 | 36,9 |
| 2 | 15 | 27,8 | 5 | 27,5 |
| 3 | 11 | 26 | 6 | 16 |
| 4 | 6 | 24 | 5 | 12 |
| 7 | 2 | 8 | 6 | 8 |

### Exemple 5

On effectue l'incubation dans les mêmes conditions que pour l'exemple 2, sauf que la levure a 8 j. Le tableau 4 ci-après donne le rendement total en FFT et ses métabolites ainsi que les rendements individuels.

**Tableau 4**

| **Rendement (%)** | **Après 1 j** | **Après 2 j** | **Après 3 j** | **Après 4 j** | **Après 7 j** |
|---|---|---|---|---|---|
| Total | 8,1 | 16,5 | 17,4 | 18,1 | 16,6 |
| FFT | 6,2 | 15 | 14 | 12,5 | 13,2 |
| Me-FFT | - | 0,5 | 0,5 | 1 | 0,5 |
| Ac-FFT | 1,9 | 1 | 1,9 | 2,6 | 0,5 |
| Di-FFT | - | - | 1 | 2 | 2,4 |

### Exemple 6

On effectue l'incubation dans les mêmes conditions que pour l'exemple 4, sauf que la levure a 8 j. Le tableau 5 ci-après donne le rendement total en FFT et ses métabolites ainsi que les rendements individuels.

**Tableau 5**

| **Rendement (%)** | **Après 1 j** | **Après 2 j** | **Après 3 j** | **Après 4 j** | **Après 7 j** |
|---|---|---|---|---|---|
| Total | 41 | 32 | 22 | 17 | 24 |
| FFT | 37 | 28 | 17 | 13 | 8 |
| Me-FFT | - | - | - | - | - |
| Ac-FFT | 2 | - | - | - | - |
| Di-FFT | 2 | 4 | 5 | 4 | 16 |

### Exemple 7

On effectue l'incubation dans les mêmes conditions que dans l'exemple 2, sauf que l'on utilise de la levure fraîche (0 j) et respectivement de 18 j. Pour effectuer l'incubation avec une levure de 18 j, on conserve la levure au réfrigérateur à 4° C. Juste avant l'utilisation, on élimine la solution de tampon phosphate par centrifugation et on la remplace par un même volume de solution tampon fraîche. Le tableau 6 ci-après donne le rendement en FFT.

**Tableau 6**

| **Rendement FFT (%)** | **Après 1 j** | **Après 2 j** | **Après 3 j** | **Après 4 j** | **Après 7 j** |
|---|---|---|---|---|---|
| Levure fraîche | 1 | 16 | 24 | - | - |
| Levure de 18 j | 13 | 22 | 22,5 | 20 | 9 |
| - : Non déterminé. | | | | | |

### Exemples 8-10

En procédant comme à l'exemple 2, on réalise l'incubation des conjugués :
**8.** Cystéine-benzaldéhyde et on obtient sa bioconversion en benzyl thiol.
**9.** 5-Méthyl-furfuryl-cystéine et on obtient sa bioconversion en 5-méthyl-furfuryl thiol et
**10.** 2-Méthyl-tétrahydrofuranyl-cystéine, ce qui conduit au 2-méthyl-tétrahydrofuran-3-thiol.

### Exemples 11-13

En procédant de manière analogue à l'exemple 2, on obtient la transformation de cystéine-furfural en FFT en l'incubant avec les micro-organismes:
**11.** Candida versatilis,
**12.** Debaromyces hansenii et
**13.** Saccharomyces bayanus.

## Revendications

1. Procédé de préparation de thiols, notamment de thiols naturels aromatisants par bioconversion, caractérisé par le fait que l'on met en présence un S-conjugué de cystéine de formule: 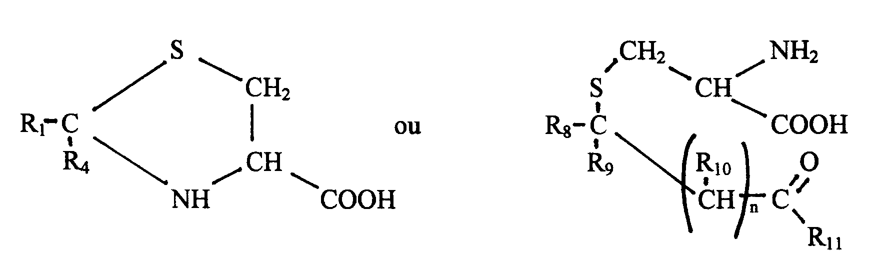 dans laquelle:
R₁ est H, C₁₋₇ -alkyl, linéaire ou branché, phényl, benzyl, 2-R₂-2-R₃-vinyl, 5-R₂-furan-2-yl, 5-R₂-thiofuran-2-yl,
R₂ et R₃ sont semblables ou différents et représentent H ou CH₃,
R₄ est H, ou forme avec R₁ un groupe 2-R₅-5-R₆-tétrahydrofuran-3-ylidène, 2-R₅-5-R₆-2,3-dihydrofuran-3-ylidène, dans lequel R₅ et R₆ sont semblables ou différents et représentent H ou CH₃,
ou R₄ est un groupe CH(OH)R₇ et R₇ représente H, C_{1₋4}-alkyl ou phényl,
R₈, R₉ et R₁₀ sont semblables ou différents et représentent H ou C₁₋₄-alkyl,
R₁₁ représente H, C₁₋₄-alkyl, phényl, C₁₋₄-alkoxy ou C₁₋₄-alkylamino et n est 0 ou 1,
et un micro-organisme utilisable en alimentation ayant une activité enzymatique de type béta-C-S-lyase.

2. Procédé selon la revendication 1, caractérisé par le fait que R₁ est méthyl, éthyl, n-propyl, isopropyl, n-butyl, isobutyl, sec-butyl, n-hexyl ou n-heptyl.

3. Procédé selon la revendication 1, caractérisé par le fait que R₈ à R₁₀ est H ou méthyl et R₁₁ méthyl, éthyl, phényl, méthoxy, éthoxy, méthylamino ou éthylamino.

4. Procédé selon la revendication 1, caractérisé par le fait que les composés préférés libérés par la béta-C-S-lyase sont choisis parmi le furfuryl thiol, le benzyl thiol, le 5-méthylfurfuryl thiol, le 2-méthylfuran-3-thiol, le 2,5-diméthylfùran-3-thiol, le 2-méthyltétrahydrofuran-3-thiol et le 3-méthyl-2-butène-1-thiol.

5. Procédé selon la revendication 1, caractérisé par le fait que le micro-organisme mis en oeuvre est la levure boulangère, Saccharomyces cerevisae.

6. Procédé selon la revendication 1, caractérisé par le fait que le micro-organisme mis en oeuvre est choisi parmi la levure de bière, les levures Candida versatilis, Debaromyces hansenii, Saccharomyces bayanus et Saccaromyces rouxii.

7. Procédé selon la revendication 1, caractérisé par le fait que le micro-organisme est un champignon filamenteux, notamment Aspergillus oryzae, Penicillium roqueforti ou Penicillium camemberti.

8. Procédé selon la revendication 5, caractérisé par le fait que la réaction d'incubation avec le micro-organisme a lieu dans des conditions d'anaérobie, pendant 5 à 72 h et de préférence pendant 12 à 48 h et à pH 6-9, et de préférence à pH 7-8, sous agitation moyenne à forte et à une température de 25 à 40° C et de préférence autour d'environ 30° C et que la concentration du substrat, c'est à dire du conjugué dans le bouillon est de 1 à 100 mMol et de préférence de 20 à 40 mMol.

9. Procédé selon la revendication 5, caractérisé par le fait que la levure boulangère est de préférence fraîche, particulièrement de 0 à 18 j, et avantageusement de 0 à 8 j.

10. Procédé selon la revendication 1, caractérisé par le fait que l'incubation comprend une addition du substrat en une ou plusieurs fois, et de préférence de manière progressive, en tenant compte de la cinétique de la réaction enzymatique, de sorte que ce dernier n'inhibe pas l'activité de l'enzyme.

11. Procédé selon la revendication 1, caractérisé par le fait que l'on augmenter la quantité de substrat et que l'on extrait les thiols volatils au fûr et à mesure de leur production, de manière à augmenter le rendement et la vitesse de la réaction enzymatique.

12. Procédé selon la revendication 1, caractérisé par le fait qu'après l'incubation, on extrait les thiols formés dans le surnageant que l'on sépare, puis on les purifie par les méthodes connues, ou on les concentre et les sèche en présence d'un support solide dans des conditions modérées, notamment à température < à 70° C sous vide.

13. Utilisation du produit obtenu par la mise en oeuvre du procédé selon l'une des revendications 1 à 12 contenant les thiols et leurs métabolites comme ingrédients aromatisants.

14. Aliment destiné à l'alimentation animale ou humaine caractérise par le fait qu'il contient un produit obtenu par le procédé selon l'une des revendications 1 à 12 ou une composition aromatique le contenant.
